Europäisches Patentamt

(19) European Patent Office

Office européen des brevets

(11) EP 0 711 172 B1

(12) EUROPÄISCHE PATENTSCHRIFT

(45) Veröffentlichungstag und Bekanntmachung des Hinweises auf die Patenterteilung:
15.01.1997 Patentblatt 1997/03

(21) Anmeldenummer: 94925408.0

(22) Anmeldetag: 28.07.1994

(51) Int. Cl.⁶: A61K 35/78

(86) Internationale Anmeldenummer:
PCT/EP94/02497

(87) Internationale Veröffentlichungsnummer:
WO 95/03816 (09.02.1995 Gazette 1995/07)

(54) **PRÄPARATE AUS CRATAEGUS-ARTEN, ARZNEIMITTEL UND IHRE VERWENDUNG ZUR VERHINDERUNG DES PLÖTZLICHEN HERZTODES SOWIE VON REPERFUSIONS-BEDINGTEN CARDIOVASKULÄREN SCHÄDIGUNGEN**

COMPOSITIONS OF CRATAEGUS SPECIES, MEDICAMENTS AND THEIR USE FOR PREVENTING SUDDEN DEATH DUE TO CARDIAC ARREST AND REPERFUSION-CAUSED CARDIOVASCULAR LESIONS

PREPARATIONS DU TYPE CRATAEGUS, MEDICAMENTS ET LEUR UTILISATION POUR PREVENIR LA MORT SUBITE PAR ARRET CARDIAQUE AINSI QUE DES LESIONS CARDIOVASCULAIRES DUES A DES REPERFUSIONS

(84) Benannte Vertragsstaaten:
AT BE CH DE DK ES FR GB GR IE IT LI LU MC NL PT SE

(30) Priorität: 29.07.1993 DE 4325532

(43) Veröffentlichungstag der Anmeldung:
15.05.1996 Patentblatt 1996/20

(73) Patentinhaber: Dr. Willmar Schwabe GmbH & Co.
D-76209 Karlsruhe (DE)

(72) Erfinder:
• CHATTERJEE, Shyam, Sunder
D-76139 Karlsruhe (DE)
• JAGGY, Hermann, Ernst, Walter
D-76229 Karlsruhe (DE)

(74) Vertreter: Vossius, Volker, Dr. et al
Dr. Volker Vossius,
Patentanwaltskanzlei - Rechtsanwaltskanzlei,
Holbeinstrasse 5
81679 München (DE)

(56) Entgegenhaltungen:
DE-A- 1 103 519          FR-A- 2 315 946
US-A- 2 799 618

• DATABASE WPI Section Ch, Week 9311, Derwent Publications Ltd., London, GB; Class A96, AN 93-086119 & CN,A,1 062 294 (XIAN BOTANICAL GARDEN) 1. Juli 1992
• PRAXIS MAGAZIN MED., Nr.1, 1994 Seiten 30 - 31 T. PETERS 'ZARTES PFLÄNZCHEN-STARKE WIRKUNG'
• GIORNALE DI GERONTOLOGIA, Bd.16, Nr.9, September 1968 Seiten 979 - 984 G. MASSONI 'L'IMPIEGO DI UN ESTRATTO DI BIANCOSPINO (CRATAEGUS) NEL TRATTAMENTO DI ALCUNE FORME DI MIOCARDIOPATIA ISCHEMICA DELL'ETA AVANZATA.'
• COMPARATIVE MEDICINE EAST AND WEST, Bd.6, Nr.2, 1978 Seiten 123 - 130 V. PETKOV ET AL. 'PHARMACOLOGICAL STUDIES ON SUBSTANCES OF PLANT ORIGIN WITH CORONARY DILATATING AND ANTIARRHYTHMIC ACTION.'
• PLANTA MEDICA, Bd.42, Nr.1, Mai 1981 Seiten 1 - 16 MITSUAKI IWAMOTO ET AL. 'KLINISCHE WIRKUNG VON CRATAEGUTT BEI HERZERKRANKUNGEN ISCHÄMISCHER UND/ODER HYPERTENSIVER GENESE'
• FORTSCHRITTE DER MEDIZIN, Bd.104, Nr.42, 13. November 1986 Seiten 57 - 60 M. O'CONOLLY ET AL. 'BEHANDLUNG DER NACHLASSENDEN HERZLEISTUNG.'

- PRAXIS MAGAZIN MED., Nr.5, 1992 Seiten 12 - 15 H. EICHSTÄDT 'CRATAEGUS-EXTRAKT HILFT DEM PATIENTEN MIT NYHA II-HERZINSUFFIZIENZ'

**Beschreibung**

Durch eine Vielzahl von klinischen Arbeiten ist belegt, daß Extrakte aus Blättern und Blüten und/oder Früchten von Crataegus-Arten beim Menschen mit nachlassender Leistungsfähigkeit des Herzens wirksam sind. Langjährige praktische ärztliche Erfahrung spricht außerdem für eine therapeutische Wirksamkeit solcher Extrakte bei instabilen funktionell-vegetativen Stenocardien sowie bei leichten Formen von bradykarden Herzrhythmusstörungen. Diese Befunde haben die Kommission E beim deutschen Bundesgesundheitsamt veranlaßt, eine Monographie zu verabschieden (s. Bundesanzeiger vom 03.01.1984) mit der Angabe folgender Indikationen:

Nachlassende Leistungsfähigkeit des Herzens entsprechend Stadien I und II der NYHA, Druck- und Beklemmungsgefühl in der Herzgegend, noch nicht digitalisbedürftiges Altersherz und leichte Formen von bradykarden Herzrhythmusstörungen.

Der bekannte therapeutische Nutzen sowie das pharmakodynamische Wirkungsspektrum dieser Crataegus-Extrakte wird bis jetzt in der Regel durch das Vorhandensein von Flavonoiden und oligomeren Procyanidinen in den Crataegus-Extrakten erklärt. Dementsprechend werden die Arzneipräparate auf die genannten Komponenten standardisiert. Einer dieser bekannten Crataegus-Extrakte (WS-1442) enthält etwa 18,5 bis 19,5% oligomere Procyanidine.

Die Erfindung beruht auf dem überraschenden Befund, daß ein durch Extraktion mit einem Gemisch aus Wasser und einem wassermischbaren organischen Lösungsmittel wie Ethanol erhaltener Extrakt aus Blättern mit Blüten von geeigneten Crataegus-Arten, wie Crataegus monogyna JAQUIN emend. LINDMAN, Crataegus laevigata (POIRET) DE CANDOLLE syn. Crataegus oxyacantha L. p. p. et duct., Crataegus azarolus L., Crataegus nigra WALDSTEIN et KITAIBEL und Crataegus pentagyna WALDSTEIN et KITAIBEL ex WILLDENOW, bei Versuchen an der Ratte mit experimentell erzeugter Ischämie

a) cardioprotektive Wirkungen gegen Herzischämie und durch Reperfusion induzierte lebensbedrohliche Schäden besitzt,

b) die bisher bekannten Wirkstoffe des Crataegus-Extrakts nicht für diese Wirkungen verantwortlich sind.

Aus diesen Wirkungen des Crataegus-Extraktes - nachstehend auch als Fraktion (a) bezeichnet - läßt sich eine therapeutische Verwendung von Crataegus-Extrakten bei der Prophylaxe und Behandlung von kurzzeitiger Herzischämie (Durchblutungsstörungen am Herzen), durch Reperfusion verursachten cardialen Schädigungen und plötzlichem Herztod sowie anderen durch Reperfusion verursachten lebensbedrohlichen pathologischen Zuständen ableiten.

Weitergehende Versuche mit drei neuen Fraktionen aus diesem Crataegus-Extrakt deuten darauf hin, daß die besonders wirksame Komponente des Crataegus-Extraktes ein Molekulargewicht größer als 3000 Dalton aufweist. Die Struktur dieser Komponente ist noch nicht bekannt.

Beschreibung der Versuche:

Getestete Substanzen:

| | |
|---|---|
| Fraktion (a) | Gesamtextrakt aus Blättern mit Blüten von Crataegus monogyna, laevigata, azarolus, nigra und pentagyna, hergestellt durch Extraktion mit 45 Gew.-% Ethanol bei 60°C, mit einem Gehalt von 18,5 bis 19,5% oligomeren Procyanidinen; |
| Fraktion (b) | Fraktion der Verbindungen mit Molekulargewichten größer als 3000 Dalton, hergestellt durch Ultrafiltration der Fraktion (a) über Membranfilter mit einer Ausschlußgrenze von 3000 Dalton; |
| Fraktion (c) | hergestellt durch flüssig-flüssig Verteilung (Gegenstrom-Extraktion) der Fraktion (a) in Butanol/Wasser; |
| Fraktion (d) | hergestellt aus der Fraktion (a), aus welcher die Flavonoide und Proanthocyanidine durch Adsorption an einem diese Verbindungen bindenden Adsorptionsmittel, insbesondere hydroxypropyliertem Polydextran, Polyamiden, Aluminiumoxid oder Aktivkohle, entfernt sind. |

Testmethode:

Angewendet wurde die von Selye [H. Selye et al., Angiology 11 (1960), 398-407] beschriebene Methode an mit Pentobarbital anaesthetisierten männlichen Sprague-Dawley Ratten mit einem Körpergewicht zwischen 250 und 350 g. Die cardiale Ischämie wurde erzeugt durch Ligatur der Coronararterie, Reperfusion wurde initiiert durch Wiedereröffnung der Ligatur nach 7minütiger Ischämie. Gemessen wurden Blutdruck und Herzfrequenz der Tiere vor und während der Ischämie und nach der Reperfusion sowie die Dauer und Natur der Herzrhythmusstörungen in der Reperfusionsphase. Unter solchen Versuchsbedingungen sterben innerhalb einer Reperfusionsperiode von 15 Minuten ca. 50% der Kontrolltiere. Todesursache sind die durch Reperfusion verursachte hypotensive Krise und das Kammerflimmern, die sofort nach Reperfusion eintreten. Dieses pharmakologische Modell ist deshalb geeignet zur Wirksamkeitsprüfung von Arzneistoffen, die den plötzlichen Herztod bzw. die durch Reperfusion verursachten cardiovaskulären Schädigungen verhindern bzw. günstig beeinflussen können.

Versuchsergebnisse:

In diesem Modell wurden der Crataegus-Gesamtextrakt, d.h. die Fraktion (a), und die Fraktionen (b), (c) und (d) getestet. In einer ersten Versuchsreihe wurde der Gesamtextrakt in einer Dosis von 100 mg/kg/Tag per os während 6 Tagen mittels Schlundsonde verabreicht. Am 7. Tag erhielten die Tiere nochmals eine orale Dosis von 50 mg/kg. Eine Stunde danach wurden die Tiere in den Versuch genommen.

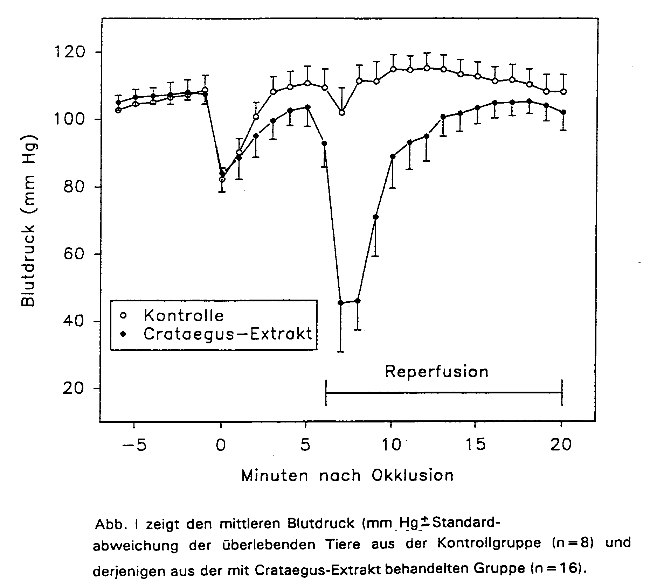

Die Versuchsergebnisse der mit dem Gesamtextrakt behandelten Tiere und der nur mit Lösungsmittel behandelten Kontrolltiere sind in Tabelle I und Abb. I zusammengefaßt. Abb. 1 zeigt den mittleren Blutdruck (mm Hg $\pm$ Standardab-weichung) der überlebenden Tiere aus der Kontrollgruppe (n = 8) und derjenigen aus der mit Crataegus-Extrakt behandelten Gruppe (n = 16). Diese Versuchsergebnisse zeigen, daß die Verabreichung von Crataegus-Gesamtextrakt die Tiere gegen die durch kurzzeitige Ischämie und Reperfusion induzierten Arrhythmien, lebensbedrohliche hypotensive Krise und eventuellen Tod schützt.

| Gruppe | Fibrillation Häufigkeit (%) | Dauer (sek.) | Tachykardie Häufigkeit (%) | Mortalität | |
|---|---|---|---|---|---|
| | | | | Dauer (sek.) | (%) |
| Kontrolle | 100 | 27,8 + 5,37 | 100 | 47,1 + 6,74 | 50 |
| Fraktion (a) | 0 | 0 | 62 | 20,0 + 4,56 | 0 |

In einer weiteren Versuchsserie wurde die Fraktion (a) in verschiedenen Dosierungen und nach unterschiedlichen Behandlungsschemata getestet. Die Versuchsergebnisse sind in Tabelle II zusammengefaßt:

Tabelle II

| Dosis (mg/kg) | Behandlungsschema | Mortalität | Schutz gegen Arrhythmien | hypotensive Krise |
|---|---|---|---|---|
| 100 | tägl. für 6 Tage + 50 mg/kg 1 Std. vor Versuch | + + + | + + + | + + + |
| 100 | 1 Tag vor + 50 mg/kg 1 Std. vor Versuch | + + | + + + | - |
| 100 | 1 Tag vor + 100 mg/kg 1 Std. vor Versuch | + + + | + + + | + |
| 100 | 1 Std. vor Versuch | + | + + | - |
| 50 | 1 Std. vor Versuch | + | + | - |
| 25 | 1 Std. vor Versuch | - | - | - |
| 100 | 4 Std. vor Versuch | + | + + | - |
| 100 | 8 Std. vor Versuch | - | + | - |

Diese Versuchsergebnisse zeigen, daß die maximalen Effekte der Fraktion (a) gegen Herzischämie und durch Reperfusion induzierte Schäden nur nach langfristiger Verabreichung der Fraktion (a) zu erzielen sind und daß nach Verabreichung von 2 x 100 mg/kg der Fraktion (a) (1 Tag + 1 Stunde vor Ischämie) nur ein kurz anhaltender aber sicherer Schutzeffekt zu erwarten ist.

In weiteren Versuchen, in welchen die Fraktion (a) in verschiedenen Dosierungen 15 Minuten vor Versuchsbeginn intravenös appliziert wurde, waren keine sicheren Effekte der Fraktion (a) feststellbar.

Die beschriebenen Fraktionen wurden danach in geeigneten Dosierungen entsprechend den jeweiligen Ausbeuten bezogen auf den Gesamtextrakt in ähnlicher Weise getestet.

Tabelle III

| Vergleich der Wirkungen des Gesamtextraktes und verschiedener Fraktionen. | | | |
|---|---|---|---|
| Fraktion (Dosis) | Mortalität | Schutz gegen Arhythmien | hypotensive Krise |
| Fraktion (a) (100 mg/kg) | + + + | + + + | + |
| Fraktion (b), Komponenten mit Molekulargewicht über 3000 Dalton aus Ultrafiltration (20 mg/kg) | + + + | + + + | + + |
| Fraktion (c) mit Molekulargewicht unter 3000 Dalton aus Filtrat der Ultrafiltration (80 mg/kg) | + + | + | - |
| Fraktion (d) ohne Flavonoide und Proanthocyanidine (50 mg/kg) | + + + | + + + | + + |

Ein Vergleich der Wirkungen der Fraktionen in Tab. III zeigt eindeutig, daß die besten Wirkungen in solchen Fraktionen aus dem Extrakt zu finden sind, die Substanzen mit höherem Molekulargewicht enthalten und daß für die Wirkungen das Vorhandensein von Flavonoiden oder oligomeren Procyanidinen, die bisher als die wirksamen Komponenten von Crataegus-Extrakten betrachtet wurden, nicht notwendig ist.

Bevorzugte Arzneimittel der Erfindung zur Prophylaxe und Therapie von reperfusionsbedingten cardiovaskulären Schädigungen, Verhinderung des plötzlichen Herztodes sowie anderen durch Reperfusion verursachten lebensbedrohlichen pathologischen Zuständen enthalten demnach eine ausreichende Menge an nichtflavonoiden polymeren Verbindungen aus Crataegus-Fraktionen mit einem hohen Gehalt an diesen Wirkstoffen.

Sie können erfindungsgemäß folgendermaßen hergestellt werden:

a) Extraktion des zerkleinerten Pflanzenmaterials mit Gemischen von Wasser, Aceton, C1-C4 Alkanolen oder Gemischen von Wasser und den genannten, wassermischbaren organischen Lösungsmitteln, vorzugsweise 45 Gew.-% Ethanol (Fraktion (a));

b) Abtrennung von Verbindungen mit niedrigem Molekulargewicht aus der Fraktion (a) durch Ultrafiltration über geeignete Membranfilter mit einer Ausschlußgrenze von 3000 Dalton (Fraktion (b));

c) erhalten durch flüssig-flüssig Verteilung der Fraktion (a) in Butanol/Wasser (Fraktion (c));

d) Gelfiltration der Fraktion (a) an Polydextranen oder anderen für die Trennung mittels Molekülsiebeffektes geeigneten Gels zur Abtrennung von Flavonoiden und Proanthocyanidinen und anderen niedermolekularen Verbindungen (Fraktion (d)).

Für die Prophylaxe und Therapie von reperfusionsbedingten cardiovaskulären Schädigungen und zur Verhinderung des plötzlichen Herztodes kann die Fraktion (a), (b), (c) und (d), vorzugsweise die Fraktion (b) in allen Arzneiformen zur oralen Applikation verwendet werden, z.B. Dragees, Tabletten, Kapseln, Lösungen. Zur Herstellung dieser Arzneimittel können die Fraktionen in üblicher Weise verarbeitet werden unter Verwendung von Trägerstoffen wie Lactose, Stärke, mikrokristalline Cellulose, Magnesiumstearat und Talcum bzw. Wasser, Alkoholen, Polyäthylenglykolen, Glycerinester. Als Zusatzstoffe sind beispielsweise Konservierungsmittel, Gleitmittel, Netzmittel, Emulgatoren, Farbstoffe, Geschmackskorrigenzien und Aromastoffe einsetzbar.

Als Tagesdosen werden 50 bis 500 mg Crataegus-Gesamtextrakt (Fraktion (a)) gegeben, bei Arzneimitteln mit angereicherten Wirkstoff-Fraktionen (Fraktionen (b), (c) und (d)) ist die Dosierung entsprechend niedriger zu wählen.

Die Beispiele erläutern bevorzugte Verfahren zur Herstellung der Fraktionen.

Die Erfindung betrifft auch die Verwendung eines durch Extraktion von Blättern und Blüten von Crataegus-Arten mit Wasser, Aceton, einem $C_1$-$C_4$ Alkanol oder Gemischen von Wasser und den genannten wassermischbaren organischen Lösungsmitteln erhältlichen Gesamtextraktes zur Herstellung eines Arzneimittels zur Verhinderung des plötzlichen Herztodes sowie von reperfusionsbedingten cardiovaskulären Schädigungen und anderen reperfusionsbedingten lebensbedrohlichen pathologischen Zuständen.

Beispiel 1:

100 kg durch Mahlung zerkleinerte getrocknete Blätter und Blüten von Crataegus-Arten (handelsübliche Mischung aus ca. 77% Crataegus monogyna, ca. 2% C. laevigata, ca. 4% C. azarolus, ca. 16% C. nigra und ca. 1 % C. pentagyna) werden mit 1000 kg 45 Gew.-% Ethanol eine Stunde bei 60°C einer Wirbelextraktion mit einem Dispax-Generator[R] unterzogen. Durch kontinuierliche Zentrifugation wird der Extrakt abgetrennt. Das Pflanzenmaterial wird nochmals in der gleichen Weise mit 1000 kg 45 Gew.-% Ethanol eine Stunde bei 60°C extrahiert. Die vereinigten Extraktlösungen werden bei einem Druck von ca. 400 mbar bei 80°C konzentriert; das Extraktkonzentrat wird in einem Vakuumtrockenschrank bei 60°C bei 5-10 mbar getrocknet.

Ausbeute an Gesamt-Extrakt (Fraktion (a)):
20% bez. auf trockene Crataegus-Blätter mit Blüten. Der Extrakt enthält etwa 18,75% oligomere Procyanidine.

Beispiel 2:

200 g durch Mahlung zerkleinerte Blätter mit Blüten von Crataegus-Arten (Gemisch aus ca. 63% C. monogyna, ca. 3% C. laevigata, ca. 9% C. azarolus und ca. 25% C. nigra/pentagyna) werden mit 2 Liter 40 Gew.-% Aceton eine Stunde unter Rühren und unter Rückfluß extrahiert. Nach dem Abtrennen der Extraktlösung wird der Rückstand noch einmal mit 2 Liter 40 Gew.-% Aceton 30 Minuten unter Rühren und unter Rückfluß extrahiert. Die vereinigten Extraktlösungen werden bei 60°C Badtemperatur und einem Druck von 520-20 mbar zur Trockne eingeengt.

Ausbeute:   65,6 g Trockenextrakt Fraktion (a), entsprechend 32,8%, bezogen auf trockene Crataegus-Blätter mit Blüten.

Beispiel 3:

20 g des aus Crataegus-Blättern mit Blüten mit 40 Gew.-% Aceton hergestellten Extrakts nach Beispiel 2 werden zu 400 ml 70°C heißem Wasser unter Rühren zugegeben. Nach Zugabe von 10 g Aktivkohle wird eine Stunde gerührt. Die Aktivkohle und ungelöste Extraktbestandteile werden über eine Filterschicht abfiltriert. Das Filtrat wird bei 60°C Badtemperatur und einem Druck von 80-20 mbar zur Trockne eingeengt.

Ausbeute:   8,8 g Substanz Fraktion (a), entsprechend 44%, bezogen auf Crataegus-Extrakt.

Beispiel 4:

85 kg durch Mahlung zerkleinerte Blätter mit Blüten von Crataegus-Arten (Gemisch aus ca. 76% C. monogyna, ca. 5% C. laevigata und ca. 19% C. nigra) werden mit 600 kg Methanol in einem Umlaufextrakteur 3 Stunden auf 60°C erwärmt. Danach wird die Extraktlösung abgetrennt. Danach wird die Extraktlösung bei 60°C und einem Druck von 200-260 mbar konzentriert und im Vakuumtrockenschrank bei 60°C und einem Druck von 20 mbar getrocknet.

Ausbeute:   12,75 kg Trockenextrakt Fraktion (a), entsprechend 15%, bezogen auf trockene Crataegus-Blätter mit Blüten.

Beispiel 5:

200 g durch Mahlung zerkleinerte Blätter mit Blüten von Crataegus-Arten (Gemisch aus ca. 63% C. monogyna, ca. 3% C. laevigata, ca. 9% C. azarolus und ca. 25% C. nigra/pentagyna) werden mit 2 Liter kochendem vollentsalztem Wasser übergossen und in einem Dispergiergerät mit Messereinsätzen 15 Minuten extrahiert.
Die Extraktlösung wird durch Filtration über eine Filterschicht auf Cellulose-Basis abfiltriert.
Der Pflanzenrückstand wird noch einmal mit 2 Litern kochendem vollentsalztem Wasser 15 Minuten wirbelextrahiert.
Die Extraktlösung aus der Nachextraktion wird ebenfalls über eine Filterschicht auf Cellulose-Basis klarfiltriert. Die Extraktlösungen werden vereinigt und in einem Rotationsverdampfer bei 60°C Badtemperatur und 50 mbar Druck zur Trockne eingeengt.

Ausbeute:   52 g Fraktion (a), entsprechend 26%, bezogen auf trockene Crataegus-Blätter mit Blüten.

Beispiel 6:

20 g Extrakt aus Crataegus-Blättern mit Blüten nach Beispiel 1 werden in 50 ml 45 Vol.-% Ethanol eingerührt und auf eine Säule aufgegeben, die 200 g quervernetztes Polyvinylpyrrolidon, angeschlämmt in 45 Vol.-% Ethanol enthält. Die Durchlaufgeschwindigkeit wird eingestellt auf 40 ml/8 Min. Am Fraktionssammler werden Fraktion zu jeweils 40 ml aufgefangen. Die ersten substanzhaltigen Fraktionen werden vereinigt und in einem Rotationsverdampfer bei 60°C Badtemperatur und einem Druck von 120-50 mbar zur Trockne eingeengt.

Ausbeute:   8,9 g Wirkstoffkonzentrat (frei von Flavonverbindungen und Procyanidinen), entsprechend 44,5%, bezogen auf Extrakt.

Beispiel 7:

6,65 g des mit 40 Gew.-% Aceton nach Beispiel 2 hergestellten Extrakts aus Crataegus-Blättern und Blüten werden in 20 ml vollentsalztem Wasser unter Erwärmen auf 50°C und Behandlung mit Ultraschall dispergiert. Die so erhaltene Suspension wird auf ein Gelbett aus 100 g hydrophilem quervernetztem Dextran (Sephadex G-25 medium$^R$) in Wasser (Höhe 57 cm, Durchmesser 2,8 cm) aufgegeben. Die ersten 240 ml substanzhaltiger Säulendurchlauf werden vereinigt und in einem Rotationsverdampfer bei 60°C Badtemperatur und 50 mbar Druck zur Trockne eingeengt.

Ausbeute:    0,13 g Rückstand (frei von Flavonverbindungen und Procyanidinen), entsprechend 1,95%, bezogen auf Extrakt.

Beispiel 8:

80 g des gemäß Beispiel 1 hergestellten Gesamt-Extrakts werden mit 1,2 Liter 50 Vol.-% Ethanol 15 Minuten bei 40°C gerührt. Die ungelösten Extraktbestandteile werden abzentrifugiert, der Überstand wird über eine Glasfritte-D3 klarfiltriert. Die Lösung wird über eine Spiralpatrone für Ultrafiltration auf Basis regenerierter Cellulose (Amicon$^R$ Spiral Ultrafiltration Cartridge Type S1Y3) mit einer Ausschlußgrenze von 3000 Dalton bei einem Druck von 2,5 bar ultrafiltriert. Nach dem Konzentrieren des Rententats auf 400 ml wird viermal mit 700 ml 50 Vol.-% Ethanol nachgewaschen. die danach erhaltenen 200 ml Retentat werden abgelassen und die Filterpatrone wird mit 1 Liter 70 Vol.-% Ethanol nachgespült. Das Retentat und die Spüllösung werden in einem Rotationsverdampfer bei 60°C und 40 mbar auf ca. 200 ml konzentriert, wobei das Ethanol abdestilliert wird. Das Konzentrat wird tiefgefroren in einem Ethanol/Trockeneis-Bad bei -40°C und gefriergetrocknet bei 0,22 mbar.

Ausbeute:    15,35 g der Fraktion (b) entsprechend 19,19% bezogen auf Gesamtextrakt.

Beispiel 9:

a) 100 g des gemäß Beispiel 1 hergestellten Gesamt-Extrakts werden unter Erwärmen auf 50°C in 1 Liter vollentsalztem Wasser gelöst. Nach dem Abkühlen auf Raumtemperatur wird die wäßrige Lösung fünfmal mit jeweils 400 ml wassergesättigtem n-Butanol ausgeschüttelt. Die Wasserphase wird aufbewahrt und in der Stufe b) weiterverarbeitet. Die Butanolphasen werden vereinigt. Danach wird die erhaltene Butanollösung einmal mit 500 ml vollentsalztem Wasser rückgewaschen. Die wäßrige Lösung wird aufbewahrt und in der Stufe b) weiter verarbeitet. Die Butanollösung wird sodann im Vakuum bei 60°C / 30 mbar konzentriert. Unter Zugabe von Wasser wird dabei das n-Butanol abdestilliert. Der Rückstand wird im Vakuum bei 60°C / 17 mbar getrocknet. Auswaage an n-Butanol Fraktion: 29,5 g.
b) Die in Stufe a) erhaltene Wasserphase sowie die beim Zurückwaschen der Butanollösung erhaltene wäßrige Lösung wird unter Vakuum bei 60°C / 55 mbar konzentriert und bei 17 mbar eingedampft. Die Nachtrocknung im Vakuumtrockenschrank bei 60°C / 20 mbar ergibt 71,4 g Fraktion (c) als Rückstand aus der Wasserphase, entsprechend 71,4% bezogen auf Gesamt-Extrakt.
In der n-Butanol-Fraktion lassen sich mittels Dünnschichtchromatographie in dem in Beispiel 4 beschriebenen System Chlorophyll, die Flavonverbindungen und ein Teil der oligomeren Procyanidine nachweisen.
Die Wasser-Fraktion (Fraktion (c)) enthält Procyanidine und noch unbekannte Wirkstoffe.

Beispiel 10:

50 g Gesamtextrakt aus Crataegus-Blättern mit Blüten wird mit 200 ml vollentsalztem Wasser bei 50°C gerührt. Die ungelöste Bestandteile enthaltende Suspension wird auf ein Gelbett aus 1 kg Sephadex-LH 20$^R$ (hydroxypropylierts quervernetztes Polydextran) - aufgeschlämmt in Wasser in einer Glassäule mit 7,4 cm Durchmesser und einer Füllhöhe von 100 cm - aufgegeben. Die Säule wird mit 5 Liter vollentsalztem Wasser eluiert. Die Durchläufe werden mittels Dünnschichtchromatographie auf Kieselgel-Platten im Fließmittel Ethylacetat/Ameisensäure/Essigsäure/Wasser 100 + 11 + 11 + 27 Volumenteilen auf Abwesenheit der für Crataegus typischen Flavonglykoside von Apigenin-Typ (Vitexin, Vitexin-2"-O-rhamnosid usw.) und Flavonglykoside des Quercetins (Hyperosid = Quercetin-3-O-galactosid, Rutin = Quercetin-3-O-rutinosid usw.) geprüft. Die Fraktionen nach 1 Liter Vorlauf bis 5 Liter Durchlauf werden vereinigt und in einem Rotationsverdampfer bei 60°C C/20 mbar zur Trockne eingeengt.

Ausbeute:    25,36 g Fraktion (d) entsprechend 50,72% bez. auf Gesamtextrakt.

EP 0 711 172 B1

Beispiel 11:

Tabletten mit 50 mg Crataegus-Gesamtextrakt (Fraktion (a))

50 g Fraktion (a)
50 g Aerosil
100 g Lactose
25 g mirokristalline Cellulose
24 g Maisstärke
1 g Magnesiumstearat

Die ersten 5 Bestandteile werden gemischt, granuliert und nach Zugabe von Magnesiumstearat auf einer Tablettiermaschine zu Tabletten zu 250 mg verpreßt.

Beispiel 12:

Kapseln mit 50 mg Crataegus-Extrakt, Fraktion (b)

30 g Fraktion (b)
120 g Lactose
50 g hochdisperse Kieselsäure

Die Bestandteile werden homogen gemischt und in üblicher Weise zu Kapseln mit einem Füllgewicht von 200 mg verarbeitet.

Beispiel 13:

| Überzogene Tabletten | | |
|---|---|---|
| Fraktion (d) | | 40,00 mg |
| mikrokristalline Cellulose | | 100,00 mg |
| Lactose | | 60,00 mg |
| kolloidale Kieselsäure | | 50,00 mg |
| Talcum (im Kern) | | 4,50 mg |
| Magnesiumstearat | | 0,50 mg |
| Hydroxpropylmethylcellulose | | 12,00 mg |
| Talcum (in der Hülle) | | 0,50 mg |
| Gewicht einer überzogenen Tablette | ca. | 267,50 mg |

Beispiel 14:

Lösung

40 g Fraktion (c)
10 g Aromaessenz
5 g Natriumsaccharinat
460 g Ethanol
475 g gereinigtes Wasser

8

# EP 0 711 172 B1

**Patentansprüche**

1. Präparat (b) aus Crataegus, erhältlich durch Extraktion von Blättern mit Blüten von Crataegus-Arten mit 45 Gew.-% Ethanol bei 60°C und Ultrafiltration des erhaltenen Gesamtextraktes (Fraktion (a)) über Membranfilter mit einer Ausschlußgrenze von 3000 Dalton.

2. Präparat (c) aus Crataegus, erhältlich durch Extraktion von Blättern mit Blüten von Crataegus-Arten mit 45 Gew.-% Ethanol bei 60°C und Abtrennung von niedermolekularen Verbindungen durch flüssig-flüssig Verteilung des erhaltenen Gesamtextraktes (Fraktion (a)) in Butanol/Wasser.

3. Präparat (d) aus Crataegus, erhältlich durch Extraktion von Blättern mit Blüten von Crataegus-Arten mit 45 Gew.-% Ethanol bei 60°C und Gelfiltration des erhaltenen Gesamtextraktes (Fraktion (a)) zur Abtrennung von Flavonoiden und Proanthocyanidinen.

4. Präparat (d) aus Crataegus, erhältlich durch Extraktion von Blättern mit Blüten von Crataegus-Arten mit 45 Gew.-% Ethanol bei 60°C und Adsorption der im erhaltenen Gesamtextrakt (Fraktion (a)) enthaltenen Flavonoide und Procyanidine an ein diese Komponenten bindendes Adsorptionsmittel.

5. Arzneimittel, gekennzeichnet durch ein Gehalt an einem Präparat nach einem der Ansprüche 1 bis 4.

6. Arzneimittel nach Anspruch 5 zur Verhinderung des plötzlichen Herztodes sowie von reperfusionsbedingten cardiovaskulären Schädigungen und anderen reperfusionsbedingten lebensbedrohlichen pathologischen Zuständen.

7. Verwendung eines durch Extraktion von Blättern und Blüten von Crataegus-Arten mit Wasser, Aceton, einem $C_1$-$C_4$ Alkanol oder Gemischen von Wasser und den genannten wassermischbaren organischen Lösungsmitteln erhältlichen Gesamtextraktes zur Herstellung eines Arzneimittels zur Verhinderung des plötzlichen Herztodes sowie von reperfusionsbedingten cardiovaskulären Schädigungen und anderen reperfusionsbedingten lebensbedrohlichen pathologischen Zuständen.

**Claims**

1. Preparation (b) from Crataegus, obtainable by extraction of leaves with flowers of Crataegus species with 45 weight percent ethanol at 60° C and ultrafiltration of the obtained total extract (fraction (a)) through membrane filter with a cut-off limit of 3000 Dalton.

2. Preparation (c) from Crataegus, obtainable by extraction of leaves with flowers of Crataegus species with 45 weight percent ethanol at 60° C and separation of low molecular compounds by liquid-liquid distribution of the obtained total extract (fraction (a)) in butanol/water.

3. Preparation (d) from Crataegus, obtainable by extraction of leaves with flowers of Crataegus species with 45 weight percent ethanol at 60° C and gel filtration of the thus obtained total extract (fraction (a)) for the separation of flavonoids and proanthocyanidins.

4. Preparation (d) from Crataegus, obtainable by extraction of leaves with flowers of Crataegus species with 45 weight percent ethanol at 60° C and adsorption of the flavonoids and procyanidins present in the thus obtained total extract (fraction (a)) onto an adsorbent capable of binding these components.

5. Pharmaceutical compositions, characterised by a content of one of the preparations according to any one of claims 1 to 4.

6. Pharmaceutical compositions according to claim 5 for preventing sudden death due to cardiac arrest and reperfusion-caused cardiovascular lesions and other reperfusion-caused life-threatening pathological conditions.

7. Use of a total extract obtainable by extraction of leaves and flowers of Crataegus species with water, acetone, a $C_1$-$C_4$ alkanol or mixtures of water and said water-miscible organic solvents for the preparation of a pharmaceutical composition for preventing sudden death due to cardiac arrest and reperfusion-caused cardiovascular lesions and other reperfusion-caused life-threatening pathological conditions.

9

**Revendications**

1. Préparation (b) issue de crataegus, pouvant être obtenue par extraction à partir de feuilles avec des fleurs du genre crataegus avec de l'éthanol à 45 % en poids à 60°C et ultrafiltration de l'extrait complet obtenu (fraction (a)) sur filtre à membrane avec une limite d'exclusion de 3000 daltons.

2. Préparation (c) issue de crataegus, pouvant être obtenue par extraction à partir de feuilles avec des fleurs du genre crataegus avec de l'éthanol à 45 % en poids à 60°C et séparation de composés de faible masse moléculaire par distribution liquide-liquide de l'extrait complet obtenu (fraction (a)) dans du butanol/eau.

3. Préparation (d) issue de crataegus, pouvant être obtenue par extraction à partir de feuilles avec des fleurs du genre crataegus avec de l'éthanol à 45 % en poids à 60°C et filtration sur gel de l'extrait complet ainsi obtenu (fraction (a)) pour la séparation des flavonoïdes et des proanthocyanidines.

4. Préparation (d) issue de crataegus, pouvant être obtenue par extraction à partir de feuilles avec des fleurs du genre crataegus avec de l'éthanol à 45 % en poids à 60°C et adsorption des flavonoïdes et des procyanidines présents dans l'extrait complet ainsi obtenu (fraction (a)) sur un adsorbant apte à fixer ces composants.

5. Composition pharmaceutique, caractérisée en ce qu'elle contient une préparation selon l'une quelconque des revendications 1 à 4.

6. Composition pharmaceutique selon la revendication 5 pour prévenir la mort subite due à un arrêt cardiaque, ainsi que les lésions cardiovasculaires provoquées par une reperfusion et d'autres états pathologiques menaçant la vie provoqués par des reperfusions.

7. Utilisation d'un extrait complet pouvant être obtenu par extraction de feuilles et de fleurs du genre crataegus avec de l'eau, de l'acétone, un alcanol en $C_1$-$C_4$ ou des mélanges d'eau et desdits solvants organiques miscibles à l'eau pour la préparation d'une composition pharmaceutique pour prévenir la mort subite due à un arrêt cardiaque, ainsi que les lésions cardiovasculaires provoquées par une reperfusion et d'autres états pathologiques menaçant la vie provoqués par des reperfusions.

Abb. I zeigt den mittleren Blutdruck (mm Hg ± Standardabweichung der überlebenden Tiere aus der Kontrollgruppe (n = 8) und
derjenigen aus der mit Crataegus-Extrakt behandelten Gruppe (n = 16).